# EUROPÄISCHE PATENTANMELDUNG

(11) **EP 0 674 006 A1**
(43) Veröffentlichungstag der Anmeldung: **27.09.1995**
(21) Anmeldenummer: 94112165.9
(22) Anmeldetag: 04.08.1994
(51) Int. Cl.: C12N 15/63, A61K 48/00, C12N 15/85

(54) **Gentherapeutisches Verfahren unter Verwendung von anti-biotikaresistenzgenfreien DNA-Vektoren**

(30) Priorität: 24.03.1994 DE 4410188
(71) Anmelder: BOEHRINGER MANNHEIM GMBH, D-68305 Mannheim (DE)
(72) Erfinder: Seeber , Stefan , Dr, D - 82377 Penzberg (DE); Rüger , Rüdiger , Dr, D - 82386 Huglfing (DE); Porteous , David , Dr, Edinburgh EH4 8EH (GB)

(57) **Zusammenfassung**

Verwendung einer Vektor-DNA zur Herstellung eines Arzneimittels zur gentherapeutischen Behandlung von Säugetieren oder Menschen, wobei die Vektor-DNA eine Modulation, Korrektur oder Aktivierung der Expression eines endogenen Gens oder die Expression eines durch die Vektor-DNA in die Zellen des Säugetieres oder des Menschen eingeführten Gens bewirkt, dadurch gekennzeichnet, daß die Vektornukleinsäure kein aktives oder im Humanbereich relevantes Antibiotikaresistenzgen enthält. Derartige Vektor-DNA sind insbesondere geeignet zur gentherapeutischen Behandlung der Atmungswege, des Verdauungstrakts und auf der Haut.

## Beschreibung

Die Erfindung betrifft die Verwendung von antibiotikaresistenzgenfreier Vektor-DNA in der Gentherapie sowie die Verwendung dieser Vektoren zur Herstellung von Arzneimitteln für die Gentherapie.

Gentherapie von somatischen Zellen kann beispielsweise unter Verwendung von retroviralen Vektoren, anderen viralen Vektoren oder durch nichtviralen Gentransfer erfolgen (zur Übersicht vgl. T. Friedmann, Science 244 (1989), 1275, Morgan 1993, RAC DATA MANAGEMENT Report, June 1993)

Gentherapeutisch geeignete Vektorsysteme sind beispielsweise Retroviren (Mulligan, R.C. (1991) in Nobel Symposium 8: Ethiology of human disease at the DNA level (Lindsten, J. and Pattersun Editors), pages 143 - 189 Raven Press), Adeno Associated Virus (McLughlin, J. Virol. 62 (1988), 1963), Vaccinia Virus, (Moss et al., Ann. Rev. Immunol. 5 (1987), 305), Bovine Papilloma Virus, (Rasmussen et al., Methods Enzymol. 139 (1987), 642) oder Viren aus der Gruppe der Herpesviren, wie Epstein Barr Virus (Margolskee et al., Mol. Cell. Biol. 8 (1988), 2937) oder Herpes simplex Virus.

Es sind auch nichtvirale Deliverysysteme bekannt. Hierzu wird üblicherweise "nackte" Nukleinsäure, vorzugsweise DNA, verwendet, oder Nukleinsäure zusammen mit einem Hilfsstoff, wie z. B. mit Transferreagenzien (Liposomen, Dendromere, Polylysin-Transferrin-Konjugate (Wagner, 1990), (Felgner et al., Proc. Natl. Acad. Sci. USA 84 (1987), 7413).

Um die für die Gentherapie verwendbare Nukleinsäure in einer therapeutischen Menge zur Verfügung zu haben, müssen diese Nukleinsäuren vor der therapeutischen Anwendung vermehrt werden. Dazu erfolgt mindestens ein Selektionsschritt, der sich ein auf der Nukleinsäure befindliches Markergen und dessen Genprodukt zunutze macht. Übliche Selektionsmarker sind beispielsweise Gene, die Resistenzen gegenüber Antibiotika wie Ampicillin, Chloramphenicol, Erythromycin, Kanamycin, Neomycin und Tetracyclin (Davies et al., Ann. Rev. Microbiol. 32 (1978) 469) vermitteln.

Es sind bereits mehrere Gentherapie-Protokolle bekannt, die sich entweder noch im Stadium von Tierexperimenten (Alton, 1993; WO 93/1224; Hyde, 1993, Debs, 1991) bzw. bereits in klinischen Studien am Patienten (Nabel, 1993, 1994) befinden. In diesen Protokollen wird meist ein auf pBR322 bzw. pUC18/19 basierender Vektor verwendet, der als bakteriellen Selektionsmarker ein Ampicillinresistenzgen trägt.

Bei Applikation von Nukleinsäuren in einer gentherapeutischen Behandlung besteht die Möglichkeit, daß im Atmungs- und Verdauungstrakt und auf der Haut vorhandene Bakterien die Nukleinsäuren aufnehmen. Wenn der Marker jedoch ein aktives Antibiotikaresistenzgen ist, könnte dadurch als unerwünschter Nebeneffekt beim Patienten eine Antibiotikaresistenz erzeugt werden. Dies ist insbesondere von Nachteil bei der gentherapeutischen Behandlung der zystischen Fibrose. Hierbei werden dem Patienten große Mengen von Vektornukleinsäure als Plasmid-DNA, oder als Aerosol unter Verwendung von Liposomen als Transferreagenz appliziert (Alton 1993).

Patienten, die an zystischer Fibrose erkrankt sind, leiden meist zusätzlich an bakteriellen Infektionen der Atmungsorgane mit beispielsweise Pseudomonas aeruginosa, Staphylococcus aureus, Haemophilus influenzae, die üblicherweise durch Gabe von Antibiotika, wie Penicillin, behandelt werden. Eine Resistenz der Patienten gegen diese Antibiotika ist demnach nachteilig.

Die bislang beschriebenen Protokolle zur CF-Gentherapie mittels CFTR-Plasmid/Liposomen-Konjugaten bzw. Veröffentlichungen von in vitro oder zu Tierversuchen verwenden auf pUC18/19 bzw. pBR322 basierende Vektoren, welche als bakteriellen Selektionsmarker das Ampicillin-Resistenzgen enthalten (Alton, 1993; WO 93/1224; Hyde, 1993).

Auch in den anderen In vivo Gentherapie-Protokollen bzw. Veröffentlichungen von In Vitro- oder Tiermodellstudien mit nackter DNA, bzw. DNA/Transfersystem-Konjugate werden die gängigen E. coli-Vektoren, basierend auf pUC- bzw. pBR-Basis mit dem Ampicillin-Resistenzgen (Nabel, 1992; Lori, 1994; Cotten, 1994; Lew, 1994 etc.) verwendet.

Gegenstand der Erfindung ist die Verwendung einer Vektor-DNA zur Herstellung eines Arzneimittels zur gentherapeutischen Behandlung von Säugetieren oder Menschen, wobei der Vektor eine Modulation, Korrektur oder Aktivierung der Expression eines endogenen Gens oder die Expression eines durch die Vektor-DNA in die Zellen des Säugetieres oder des Menschen eingeführten Gens bewirkt, dadurch gekennzeichnet, daß die Vektornukleinsäure kein aktives Antibiotikaresistenzgen enthält.

Vorzugsweise wird das Arzneimittel als Aerosol angewendet.

Besonders bevorzugt wird eine Vektor-DNA verwendet, durch die ein defektes Gen korrigiert, ein intaktes Gen eingeführt oder am korrekten Genlocus ausgetauscht werden kann. Unter einer Vektor-DNA im Sinne der Erfindung ist ein nichtvirales DNA Molekül, auf Basis eines prokaryontischen Plasmids, zu verstehen. Zusätzlich enthält dieses DNA-Molekül die in gentherapeutischen Verfahren zu übertragende DNA, vorzugsweise ein exprimierbares Gen.

Als nichtvirale DNA im Sinne der Erfindung ist zu verstehen, daß diese DNA nicht Bestandteil eines infektiösen viralen Partikels ist und kein intaktes virales Genom enthält. Die nichtvirale DNA kann jedoch virale Sequenzen, wie z. B. Regulationssequenzen (z. B. Promotor, Enhancer), Transkriptionsstopps, Replikationsursprünge oder virale Gene, wie z. B. das Thymidinkinase-Gen aus dem Herpes simplex Virus, enthalten.

Besonders bevorzugt ist die Anwendung derartiger Vektor-DNA zur Behandlung der zystischen Fibrose am Menschen. Ein hierfür geeignetes Gen ist beispielsweise in der WO 91/02796 beschrieben. Dort ist auch die Herstellung und Anwendung von Vektoren zur gentherapeutischen Behandlung der zystischen Fibrose beschrieben.

Die DNA-Vektoren sind insbesondere zu solchen gentherapeutischen Behandlungen geeignet, bei denen die Vektoren unmittelbar mit Oberflächen in Säugetieren oder Menschen in Berührung kommen. Derartige Oberflächen sind beispielsweise der Atmungs- und der Verdauungstrakt sowie die Hautoberfläche.

Ein weiterer Gegenstand der Erfindung ist eine Vektor-DNA, welche ein Gen oder Genfragment enthält, das die Aktivierung, Modulation oder Korrektur der Expression eines endogenen zystischen Fibrosegens (CFTR-Gen, cystic fibrosis transmembrane conductance regulator gene) in Säugerzellen bewirkt oder ein CFTR-Gen enthält, welches nach Transfektion von Säugerzellen mit der Vektor-DNA die Expression dieses Gens bewirkt, dadurch gekennzeichnet, daß dieser Vektor zusätzlich ein inaktiviertes Antibiotikaresistenzgen oder ein Gen, welches für einen auxotrophen Marker codiert, enthält.

Erfindungsgemäß geeignete Nukleinsäuren können vorzugsweise nach folgenden Verfahren hergestellt werden:

### 1. Plasmide mit neutralem Marker:

- Herstellbar durch Deletion des Resistenzgens (z. B. Amp) in Vektoren (z. B. pUC 18 oder pBR 322) und Einfügung eines neutralen Markers, der keine Antibiotikaresistenz vermittelt.
- Die Vektoren tragen als neutralen Marker z. B. das lacZ-Genfragment aus pUC18, das über α-Komplementation im entsprechenden E.coli-Stamm, z. B. XL1-Blue, eine funktionsfähige β-Galaktosidase bildet, was an blau gefärbten Kolonien auf X-Gal-Agar erkennbar ist. Dies ermöglicht es, die Stabilität des Plasmidkonstruktes zu überprüfen. Die Fermentation erfolgt zweckmäßig in Vollmedium.

### 2. Plasmide mit einem auxotrophen Marker:

- Einfügen z. B. eines leu, thi, pro oder bio-Gens (kodieren für Leucin, Prolin oder Biotin-Synthesegen ) in einem Vektor und Transformation des erhaltenen Plasmidkonstruktes in einem E.coli-Stamm, der in dem entsprechenden leu, pro- oder bio-Gen defekt ist. Das plasmidkodierte Auxotropie-Markergen komplementiert den Defekt.
- Die Vorkultur wird unter Selektionsdruck in Minimalmedium ohne Leucin, Prolin oder Biotin angezogen werden.
- Die Hauptkultur wird zweckmäßig in Vollmedium fermentiert. Es muß allerdings vorher sichergestellt werden, daß die Plasmidkonstrukte eine ausreichende Stabilität besitzen. Im Falle einer Übertragung des Plasmides, beispielsweise auf Bakterien der Lungenflora, können diese keinen Nutzen aus dem übertragenen Auxotropie-Marker ziehen.

### 3. Antibiotika-Resistenzmarker (AB^{R}):

### Suppression von inaktivierten Antibiotika-Resistenz-Genen:

Eine Insertion von 1 bis 2 amber-Stop-Codons (UAG) an Stelle von Glutamin-Codons (bei supE) bzw. von Tyrosin-Codons (bei supF) führt zu einem frühzeitigen Translations-Stop und somit zu einem Nonsense-Genprodukt. Auf diese Weise läßt sich ein AB^{R}-Gen inaktivieren. Ein durch amber-Stop-Codon Insertionen inaktiviertes Ab^{R}-Gen wird in Gegenwart von supF-tRNAs (Einbau von Tyrosin am amber-Stop) bzw. supE (Einbau von Glutamin am amber-Stop) supprimiert, d.h. ein funktionsfähiges Genprodukt (AB^{R}) entsteht.

Es existieren 2 Möglichkeiten der Einführung von sicheren Resistenzmarkern in therapeutischen Plasmiden:
Insertion des durch amber-Stop-Codons inaktivierten AB^{R}-Gens an Stelle von Amp, z. B. in puC- oder pBR-Vektoren und Transformation des erhaltenen Plasmids in einen E.coli-Stamm der chromosomal codierte supE bzw. supF Suppressor tRNA-Gene besitzt. Dies ermöglicht eine Selektion der erhaltenen Transformanden auf Antibiotika haltigem Medium. Im Falle der Aufnahme solcher Plasmidkonstrukte durch Bakterien der Lungenflora könnten die aufgenommenen, inaktivierten AB^{R}-Gene nicht komplett abgelesen werden und vermitteln somit keine AB^{R}.

Insertion des nur 203 bp großen supF tRNA-Gens (Seed, 1983) anstelle eines bakteriellen Resistenzgens und Insertion des, durch ein amber-Stop-Codon inaktivierten, AB^{R}-Gens ins Chromosom des Produzenten E.coli-Stammes. Die plasmidcodierte supF tRNA supprimiert die chromosomal vorliegende, durch amber-Stops inaktivierte AB^{R}, was ein Wachstum des Produzenten E.coli-Stammes auf einem entsprechenden Antibiotikamedium ermöglicht. Die erhaltenen Plasmide sind ohne AB^{R}-Gene, d. h. es besteht beispielsweise keine Gefahr der Übertragung von AB^{R}-Genen auf Bakterien der Lungenflora.

### 4.

Ebenfalls bevorzugt ist es, eine Genkassette in einen E.coli spezifischen Bakteriophagen-Vektor, z. B. M13mp18, zu klonieren, der für den Menschen unbedenklich ist, kein Ab^{R}-Gen trägt und mit hoher Ausbeute als ds-DNA bereitgestellt werden kann. Im Falle der Aufnahme des Phagenvektors, beispielsweise durch Bakterien der Lungenflora, sind keinerlei negative Effekte zu erwarten.

Wesentlicher Anwendungszweck ist die verbesserte gentherapeutische Behandlung von zystischer Fibrose. Die bisher bekannten Verfahren zur Behandlung von zystischer Fibrose sind beispielsweise in der WO 91/2796 beschrieben. Dort ist auch ein für die Gentherapie geeignetes CFTR-Gen beschrieben.

Die zystische Fibrose ist eine schwerwiegende monogenetische, autosomal rezessive Erbkrankheit, die mit einer Frequenz von 1/2500 Geburten auftritt. Sie ist charakterisiert, durch einen mangelhaften Elektrolyttransport der Epithelgewebsmembranen, was zu Funktionsabnormalitäten (Dysfunktion exokriner Drüsen) im Respirationstrakt, Pankreas (vermehrte Produktion und erhöhte Viskosität des Sekretes mucöser Drüsen), Schweißdrüsen (erhöhter Elektrolytgehalt des Schweißes und damit verbundener Flüssigkeits- und Elektrolytverlust) und Gonaden führt. Die Respiratorische Insuffizienz auf Grund einer unzureichenden Sekretion von Chloridionen in den Bronchialschleim durch Zellen des Atmungsorgan-Epithels, stellt die häufigste klinische Manifestation und Todesursache bei CF Patienten dar. Das verantwortliche Gen konnte kloniert und das Genprodukt charakterisiert werden als "cyclic adenosine monophosphate" (cAMP) abhängiges Chloridionenkanalprotein (CFTR = Cystic Fibrosis Transmembrane Conductance Regulator) (WO 91/2796). Das Wissen über die Pathophysiologie der Krankheit, die Struktur und Funktion von CFTR und Mutationen im Zusammenhang mit den CFTR-Funktionsstörungen machen es heute möglich, neben den klassischen, nicht sehr wirkungsvollen Therapieansätzen, verschiedene Gentherapieansätze durchzuführen.

In angemeldeten bzw. laufenden klinischen Protokollen zur CF-Therapie werden bisher zwei Wege beschritten. Nach dem ersten Verfahren wird das CFTR-Gen mittels Inhalation von CFTR-Adenovirusvektoren angewendet. Adenoviren infizieren natürlicherweise das Lungenepithel. Mit dieser Methode wurden schon erste klinische Erfolge erzielt, aber nur für eine kurze Zeitspanne von wenigen Wochen und mit ungewünschten toxischen Nebenwirkungen (Zabner & Welsh, 1993). Die zweite Methode beinhaltet die Einbringung von CFTR-Plasmiden komplexiert mit kationischen Liposomen mittels Inhalation in den Respirationstrakt (Alton, 1993). Hierbei werden in Mäusen ca. 1 µg Plasmid-DNA / Maus verabreicht; bei Menschen werden die Plasmidgaben im Bereich von 300-500 µg Plasmid / Patient liegen, was bei einer Plasmidgröße von 8,2 kb (Alton, 1993; Whitsett, 1992) einer Zahl von ca. 5 x 10¹³ DNA-Molekülen entspricht. Diese Applikation und Dosierung ist auch erfindungsgemäß bevorzugt.

Die Lungenepithelzellen können nur einen kleinen Anteil der eingebrachten Plasmidmenge aufnehmen. Es ist zu erwarten, daß der größte Anteil des Plasmids entweder ausgeatmet oder vom Patienten verschluckt wird, daß also ein hoher Anteil in die Umgebung (Patienten in Unterdrucksicherheitsräumen) und den Magen/Darm-Trakt des Patienten gelangt.

In der Lungenflora befinden sich verschiedene Bakteriengattungen, von denen einige als opportunistische Pathogene in Erscheinung treten können, z.B. Pseudomonaden, Haemophilus, Enterobacteriaceae, Staphylokokken etc. (Manual of Clinical Microbiology, Balows, 1991).

Eine bakterielle Besiedelung des, bei CF-Patienten im Bereich der Atemwege verstärkt sezernierten, viskosen, eiweißreichen Sekrets durch Bakterien ist eine häufige Ursache schwerer Fälle von Bronchitis und Lungenentzündung. CF-Patienten sind vor allem Infektionen der Bronchien und Lungen durch Haemophilus influenzae, Pseudomonas aeruginosa und Staphylococcus aureus ausgesetzt (Cystic Fibrosis, Dodge, 1993, Fritz Simmons, 1993), weshalb sie in häufiger Folge Antibiotikabehandlungen unterzogen werden müssen.

Wichtigste Antibiotika hierfür sind Penicillin bzw. dessen Derivate, Ampicillin (H. influenzae) und Carbenicillin (P. aeruginosa, β-Lactamase sensitives Penicillinderivat, Davis 1980). Wegen weitverbreiteter Penicillinresistenzen sind bei S. aureus vor allem die β-Lactamase resistenten Penicillinderivate (Methicillin, Oxacillin, Cephalosporin sowie Chemotherapeutika) von Bedeutung.

Daneben sind bei Lungeninfektionen noch andere Erreger von Bedeutung, z.B. Streptococcus pneumoniae (=Pneumokokken), der häufigste Erreger von bakterieller Lungenentzündung und Enterobacteriaceae (z.B. Klebsiella pneumoniae), wobei besonders bei Pneumokokken Penicillin das wichtigste Therapeutikum ist (Davis, 1980).

Im Magen/Darm-Trakt sind unter anderem Enterobacteriaceae und Enterokokken vorhanden (Balows, 1991). Bei der Behandlung von Darminfektionen, welche durch Enterobacter, E. coli, Serratia und Streptococcus faecalis verursacht sind, spielen Penicilline und deren Derivate ebenfalls eine zentrale Rolle (Davis, 1980).

Bereits 1944 wurde von Avery (Avery, 1944) die Aufnahme von hochmolekularer DNA durch Pneumokokken aus dem Medium beschrieben, ein Vorgang, der als natürliche Kompetenz bezeichnet wird, in der bakteriellen Evolution eine wichtige Rolle spielt und daher im Bakterienreich weit verbreitet ist. Physiologische Transformation wurde bei den Gattungen Haemophilus, Streptococcus, Staphylococcus, Neisseria, Bacillus and Acinetobacter (Davis, 1980) beobachtet. Damit können auch Pseudomonaden, bei denen der horizontale Gentransfer weit verbreitet ist, hochmolekulare DNA aus dem Medium aufnehmen.

Bakterien der natürlichen Flora des Menschen (Respirationstrakt, Magen/Darmtrakt, Haut, Schleimhäute, Auge etc.) sind damit in der Lage, Plasmid-DNA aufzunehmen. Durch rekombinatorische Ereignisse kann die aufgenommene DNA in die zelleigene DNA (Chromosom, Plasmide) integrieren und dabei unter die Kontrolle eines wirtseigenen Promotors geraten, d.h. exprimiert werden.

Bei der Verabreichung von ca. 300 - 500 µg Plasmid-DNA / CF-Patient (entspricht bei einer Plasmidgröße von 8,2 kb ca. 5 x 10¹³ Molekülen; Alton 1993) besteht die Gefahr, daß unter den Bakterien der Lungenflora, aber auch der anderen Körperregionen Antibiotika-resistente Keime entstehen. Wie bereits ausgeführt, ist gerade bei CF-Patienten, die besonders unter bakteriellen Lungeninfektionen leiden und ständig mit Antibiotika cotherapiert werden müssen, eine Erzeugung von Antibiotika-Resistenzen, im besonderen Maße einer Ampicillin-Resistenz (β-Lactamase) verhängnisvoll, besonders, da die bisher verwendeten Gentherapieverfahren noch keine vollständige Heilung bzw. keine persistierende Korrektur bewirken.

Es ist außerdem nicht auszuschließen, daß das mit dem CFTR-Plasmid eingebrachte Ampicillin-Resistenzgen in die Patienten-DNA integriert, dort unter der Kontrolle eines zelleigenen Promotors exprimiert und das Genprodukt aktiv oder auch passiv (z.B. Zellyse bei Entzündungsreaktionen) ausgeschleust wird Die lokal freigesetzte β-Lactamase könnte auch im Falle einer allgemeinen bakteriellen Infektion eine Penicillin-Therapie behindern.

Vorteilhaft ist die erfindungsgemäße Verwendung von antibiotikaresistenzgenfreien DNA-Vektoren auch bei der gentherapeutischen Behandlungen von AIDS-(Lori & Gallo, 1994) bzw. Krebs-Patienten (Nabel, 1993), da in beiden Fällen die Patienten in der Regel durch das Krankheitsbild an sich (bei AIDS) bzw. durch Chemotherapeutika-Therapien oder Radiotherapien (bei Krebs) immunsupprimiert sind. Bei diesen Patienten können bakterielle Infektionen durch Antibiotika-Behandlung verhindert bzw. unter Kontrolle gebracht werden.

Neben der Lunge bzw. dem Respirationstrakt müssen auch gentherapeutische Ansätze zur Behandlung anderer Gewebe unter den oben erwähnten Gesichtspunkten betrachtet werden:
Muskelgewebe: Gentherapeutische Plasmide können z.B. zur In Vivo Vaccinierung direkt ins Muskelgewebe (Ulmer, 1993; Davis, 1993; Lew, 1994) oder in Tumore (Immunstimulation zur Tumorvaccinierung; Nabel, 1993; San, 1993) injiziert werden. Therapeutische Plasmide wurden dazu bislang in geringen Dosen injiziert, weshalb die injizierte Plasmid-DNA nur um den Stichkanal lokalisiert blieb. Um systemische Reaktionen zu erhalten, ist eine höhere Dosierung bzw. eine systemische Verabreichung der therapeutischen Plasmide nicht zu umgehen. Dies hat dann eine Verbreitung der Plasmid-DNA im Blutsystem zur Folge.

Blutsystem: Zur Gentherapie in der Leber können Konjugate von Plasmid-DNA/Polylysin/Lebertargeting-Gruppen (Chiou, 1994) intravenös verabreicht werden. Dies hat eine Ausbreitung der Plasmid-DNA im Blutsystem zur Folge.

In beiden Fällen (Muskelgewebe, Blutsystem) könnten die eingebrachten Plasmide an Bakterienherde gelangen, von diesen Bakterien aufgenommen werden und die Antibiotika-Therapie im Falle des Ausbruchs einer Infektion behindern.

Darm: Zur Gentherapie von Colon-Cancer können Konjugate aus therapeutischem Plasmid (z.B. mit Tumor Suppressor-Gen) direkt in den Darm eingebracht werden (Arenas, 1994). Ebenso wird über Gene-Delivery z.B. von Genen, welche für den LDL-Rezeptor kodieren, über die Darmschleimhaut nachgedacht. Eingebrachte Plasmide können das Antibiotika-Resistenzgen auf die Bakterien des Magen/Darm-Traktes übertragen.

Haut: Plasmid-DNA kann direkt als Konjugat mit Liposomen in Hautzellen, z.B. zur Gentherapie von Melanom oder Hämophilie B (Faktor IX, X) aufgenommen werden (Alexander, 1994). Auf diese Weise können Bakterien der Hautflora Antibiotika-Resistenzen erhalten.

Auge: Persistierende Virusinfektionen des Auge können durch Gentherapie mittels therapeutischen Plasmiden therapiert werden, was die Gefahr der Übertragung von Antibiotika-Resistenzen auf die Bakterien der Augenflora birgt.

In einer weiteren bevorzugten Ausführungsform der Erfindung wird eine Vektor-DNA verwendet, welche ein Gen oder Genfragment enthält, das die Modulation, Aktivierung oder Korrektur eines endogenen zystischen Fibrosegens in menschlichen Zellen bewirkt, oder welche ein zystisches Fibrosegen enthält, dadurch gekennzeichnet, daß diese Vektor-DNA als Antibiotikaresistenzgen ein Resistenzgen für Hygromycin, Chloramphenicol, Spectinomycin, Blasticidin S, Phleomycin, Bleomycin, Puromycin, Apramycin oder Tetronasin enthält. Diese Antibiotikamarker haben im Humanbereich nur limitierte klinische Relevanz, da die entsprechenden Antibiotika am Menschen üblicherweise nicht angewendet werden. (Beispielsweise ist Hygromycin, Hyg^{R}; Veterinärmedizin, nicht in der "Roten Liste"; Rote Liste, 1994; Gritz, 1983 oder Chloramphenicol-Resistenz Cm^{R}; nur vereinzelte, sehr spezielle Anwendung bei lebensbedrohenden Infektionen; Rote Liste, 1994).

Die Plasmid-Amplifikation kann vorzugsweise auch mit Erythromycin oder Spectinomycin durchgeführt werden.

### Weiterhin bevorzugt sind:

### a) Vektoren mit Hygromycinresistenzmarker (s.o.; Hygromycin B Phosphotransferase-Gen)

Hygromycin B hat für den Menschen keine klinische Relevanz. Es wird nur in der Veterinärmedizin verwendet. Die Verwendung eines Hyg^{R}-Markergens in einem therapeutischen Plasmid ergibt, bei Übertragung des Plasmids auf Bakterien der natürlichen Körperflora, keine Resistenzen gegen klinisch relevante Antibiotika.

### b) Vektoren mit Spectinomycinresistenzmarker (O-Adenyltransferase-Gen)

Spectinomycin spielt als Antibiotikum nur eine untergeordnete Rolle. Es ist nur 1 Spectinomycinpräperat gegen Gonokokkeninfektionen zugelassen (Rote Liste). Die Verwendung eines Spectinomycin-Markergens in einem therapeutischen Plasmid ergibt bei Übertragung des Plasmids auf Bakterien der natürlichen Körperflora keine Resistenzen gegen klinisch relevante Antibiotika.

### c) Vektoren mit Chloramphenicolresistenzmarker (Chloramphenicol-acetyl-transferase-Gen)

Chlorampenicol hat nur bedingte klinische Relevanz. Es wird nur in einzelnen, sehr speziellen Fällen, bei lebensbedrohenden Infektionen z.B. Rickettsiosen, Thyphus, Paratyphus, Salmonella-Sepsis und Meningitis verwendet. Die Verwendung eines Cm^{R}-Markergens in einem therapeutischen Plasmid ergibt, bei Übertragung des Plasmids auf Bakterien der natürlichen Lungenflora, keine Resistenzen gegen klinisch relevante Antibiotika.

### d) Vektoren mit Blasticidin S Resistenzmarker (BS-Deaminase)

BS findet als mikrobielles Fungizid Anwendung (Kamakura, 1987). Es hat keinerlei Relevanz als therapeutisches Antibiotikum beim Menschen. BS wirkt sowohl in Bakterien als auch Eukaryonten, indem es die Protein Biosynthese hemmt. Das entsprechende Resistenzgen, die BS-Deaminase (eine Nukleosid Aminohydrolase) konnte aus Bacillus cereus K55-S1 in E. coli und B. subtilis kloniert und exprimiert werden. E. coli-Transformanten sind gegen eine Dosis von 200 µg/ml resistent (Kamakura, 1987).

Das BS Resistenzgen kann daher als sicheres Selektionsmarkergen in einem therapeutischen Plasmid verwendet werden. Bei Übertragung auf Bakterien der menschlichen Körperflora sind keinerlei Probleme mit entstehenden Resistenzen zu erwarten, da BS zur Therapie im Menschen nicht zugelassen ist.

### e) Vektoren mit Phleomycin/Bleomycin Resistenzmarker

Bleomycin und Phleomycin wirken sowohl auf Prokaryonten als auch auf Eukaryonten, indem sie Strangbrüche an spezifischen Sites der Zell-DNA hervorrufen. Lediglich Bleomycin hat trotz seiner hohen Lungentoxizität klinische Relevanz, doch nicht als Antibiotikum, sondern trotz seiner hohen Lungentoxizität als Cytostatikum bei der Therapie verschiedener humaner Krebsarten. Es konnten verschiedene Ble^{R}-Resistenzgene in E. coli kloniert und exprimiert werden, welche vermutlich durch Bindung an Bleomycin/Phleomycin ein Schneiden der DNA verhindern. E. coli-Transformanten konnten auf Bleomycin bzw. Phleomycin haltigem Medium (1 µg/ml Phleomycin) selektioniert werden (Mulsant, 1988).

Die ble^{R}-Gene sind daher sichere Selektionsmarkergene für therapeutische Plasmide. Bei Übertragung auf Bakterien der menschlichen Körperflora sind keinerlei Probleme mit entstehenden Resistenzen zu erwarten, da Bleomycin/Phleomycin als Antibiotika zur Therapie im Menschen nicht zugelassen ist.

### f) Vektoren mit Puromycin Resistenzmarker

Puromycin ist ein Aminoglycosidantibiotikum, welches die Peptidkettenverlängerung während der Translation inhibiert, indem es mit der A-site der großen ribosomalen Untereinheit prokaryotischer (70S) als auch eukaryotischer (80S) Ribosomen interagiert. Puromycin kann als dominanter Marker auch in Eukaryonten verwendet werden, hat daher wegen seiner hohen Toxizität als therapeutisches Antibiotikum in der Humanmedizin keinerlei Relevanz. Puromycin wird produziert durch Streptomyces alboniger, aus welchem auch das Puromycin-Resistenzgen (Pac^{R}-Gen, eine Puromycin N-acetyl Transferase) in Streptomyces lividans und E. coli kloniert und exprimiert werden konnte (Vara, 1985).

Das Pac^{R}-Gen kann als sicheres Selektionsmarkergen für therapeutische Plasmide angesehen werden. Bei Übertragung auf Bakterien der menschlichen Körperflora sind keinerlei Probleme mit entstehenden Resistenzen zu erwarten, da Puromycin als Antibiotikum zur Therapie im Menschen nicht zugelassen ist.

### g) Vektoren mit Apramycin Resistenzmarker

Apramycin ist ein Aminoglycosidantibiotikum. Es hat keinerlei klinische Relevanz. Es wurde in E. coli ein Apramycin-Resistenzplasmid beschrieben das ein Aminoglycosid-Acetyltransferase-Gen trägt (Hunter, 1992). Das Apramycin-Resistenzgen kann als sicheres Selektionsmarkergen für therapeutische Plasmide angesehen werden. Bei Übertragung auf Bakterien der menschlichen Körperflora sind keinerlei Probleme mit entstehenden Resistenzen zu erwarten, da Apramycin als Antibiotikum zur Therapie im Menschen nicht zugelassen ist.

### h) Vektoren mit Tetronasin Resistenzmarker

Tetronasin (Tn) gehört zu den Ionophoren-bildenden Antibiotika. Es hat lediglich in der Veterinärmedizin als Futtermittel-Antibiotikum Relevanz, ist aber in der Humanmedizin nicht zugelassen. Es wurde ein Resistenzgen gegen Tetronasin aus Bacteroides ruminicola isoliert (Newbold, 1992). Das Tetronasin-Resistenzgen kann als sicheres Selektionsmarkergen für therapeutische Plasmide angesehen werden. Bei Übertragung auf Bakterien der menschlichen Körperflora sind keinerlei Probleme mit entstehenden Resistenzen zu erwarten, da Tetronasin als Antibiotikum zur Therapie im Menschen nicht zugelassen ist.

Die nachfolgenden Beispiele erläutern die Erfindung weiter. Eine ausführliche Beschreibung der experimentellen Bedingungen ist in J. Sambrook, Molecular Cloning, Second Edition (1989) Cold Spring Harbor Laboratory Press, New York enthalten.

### Beispiel 1

### Bestimmung der Aufnahmerate von Plasmiden und Erzeugung von Antibiotikaresistenzen durch Bakterien der humanen Lungen- und Darmflora

Es soll gezeigt werden, daß E. coli Plasmid-DNA z.B. pUC18 bzw. Derivate davon von Bakterien anderer Gattungen aufgenommen und das Ampicillinresistenzgen exprimiert werden kann, was zu ampicillinresistenten Klonen führt. Die Wahrscheinlichkeit der Entstehung ampicillinresistenter Keime wird bestimmt durch die Aufnahmerate der Plasmid-DNA aus dem Substrat und der Einbaurate des Ampicillinresistenzgens durch nichthomologe Rekombination in die chromosomale DNA des Wirtsbakteriums. Plasmide mit alternativen Markern bzw. durch Stop-Codons inaktivierten Markern (siehe oben) rufen keine Resistenzbildung mehr hervor.

Die Untersuchungen werden an folgenden Bakterienarten durchgeführt:
Gram-negative:
Haemophilus influenzae
Pseudomonas aeruginosa
Klebsiella pneumoniae
Escherichia coli (WT)
Gram-positive:
Staphylococcus aureus
Streptococcus pneumoniae
Dazu werden verschiedene Mengen Plasmid-DNA unter verschiedenen Bedingungen zu Kulturen der oben genannten Organismen zugegeben.

Es werden Flüssigkulturen in Nährmedium, unter Verwendung von isotonischem Puffer (NaCl, Mg²⁺, Ca²⁺) durchgeführt. Diesen Kulturen wird Plasmid-DNA (z.B. pUC18; mit Ap^{R}-Gen) verschiedener Konzentration zugegeben. Nach Inkubation über mehrere Stunden unter Schütteln, wird Antibiotikum zugegeben. Es wird weiter inkubiert, ausplattiert auf einem antibiotikahaltigen Agar und eine Verdünnungsreihe auf nicht antibiotikahaltigem Agar durchgeführt. Die Lebendkeimzahl wird bestimmt nach Inkubation durch Auszählen und Identifizieren bzw. Charakterisieren der erhaltenen Kolonien.

Alternativ wird eine definierte Plasmidmenge und Bakterien definierter Keimzahl auf Nähragar ausplattiert und einige Stunden ihkubiert, Antibiotikum durch Aufsprühen zugegeben und die erhaltenen Kolonien ausgezahlt und identifiziert bzw. charakterisiert. Ebenso kann eine definierte Plasmidmenge und Bakterien definierter Keimzahl auf einem Nylonfilter aufgetragen werden, die Filter einige Stunden auf Nähragar inkubiert werden. Anschließend werden die Filter auf antibiotikahaltigem Nähragar umgesetzt, inkubiert und die erhaltenen Kolonien ausgezählt und identifiziert bzw. charakterisiert.

### Beispiel 2

### Konstruktion von Sicherheitsvektoren auf Basis von pUC18, die keinen Ampicillin-Selektionsmarker mehr besitzen.

### Austausch des Ampicillin-Resistenzgens (Amp^{R}) von pUC18

### 1. gegen das Hygromycin-Resistenzgen (Hyg^{R}) aus pHPH0

Die DNA-Sequenz des Hygromycin-Resistenzgens (Gritz, 1983) ist in der EMBL-Datenbank unter EMBL1.PJHPH gespeichert.
- Amplifikation von pUC18 ohne dem Amp^{R}-Leserahmen mittels PCR
   Primer 1 (pUC-U1): bp 2489-2513 von PUC18.seq; (SEQ ID NO:1) 5'CGAGTGAAGACACCATGGTCTTCCTTTTTCAATATTATTGAAG ^{*)}
   Primer 2 (pUC-L): bp 1628 - 1606 von PUC18.seq; (SEQ ID NO:2) 5'GGACTAGATCTGCTAGCTAACTGTCAGACCAAGTTTACTC
- Restriktion des Amplifikats mit NcoI / NheI
- Präparatives Agarosegel + Ausschneiden des pUC18-Amplifikats
- Ligation mit dem Hyg^{R}-Amplifikat
- Amplifikation des Hyg^{R}-Leserahmens aus pHDH0 mittels PCR
   Primer 3 (HygR-U): bp 260 - 278 von PHPH0.seq; (SEQ ID NO:3) 5'GCTGTAGATCTCATGAAAAAGCCTGAACTCACCGCGACG
   Primer 4 (HygR-L): bp 1257 - 1276 von PHPH0.seq; (SEQ ID NO:4) 5'CGACAGATCTGCTAGCTCATTATTCCTTTGCCCTCGGACG
- Restriktion des Amplifikats mit BspHI / NheI
- Präparatives Agarosegel + Auschneiden des Hyg^{R}-Amplifikats
- Ligation mit dem pUC18-Amplifikat (s.o.)
- Transformation in E. coli XL1-Blue und Selektion auf Hygromycin B (125 µg/ml) haltigem Medium
- Isolierung der resultierenden Plasmid-DNA pUC18-HygR
- Sequenzanalyse von pUC18-HygR

*) Unterstrichener Bereich der Primer bindet an das DNA-Template

### 2. Einfügen von Stop-Codons in den Leserahmen von Hyg^{R}

Es wurden mittels PCR amber-Stops (UAG) in den Leserahmen des Hyg^{R}-Gens von pUC18-HygR eingeführt.

SupF supprimiert besser als supE; amber-Stops, denen ein G oder A nachfolgt werden besser supprimiert als wenn ein T oder C folgt. Daher wurden an zwei günstig gelegenen (nahe einer singulären Restriktions-Site) Tyrosin-Codons (TAT oder TAC), mittels mutierter PCR-Primer, Punktmutationen eingeführt.
1. Tyr-Codon an Position 640 - 642 (EMBL1.PJHPH) von TAC zu TAG (= amber-Stop)
   Primer 8 (pUC18-HygR-Mut640) (SEQ ID NO:8)
   - 5'GCAGATCTCGGACCGCAAGGAATCGGTCAATAGACTACATGGCG
   - PCR mit Universal-Sequencing-Primer gegen pUC18-HygR Template
   - Restriktion des Amplifikats und pUC18-HygR mit RsrII / NheI
   - Präparatives Agarosegel; Elution des 600 bp großen Amplifikats und des größeren Fragments von pUC18-HygR
   - Ligation von der beiden Fragmente
   - Transformation in E. coli ER1458 (supF) und Selektion auf Hygromycin B (125 µg/ml) haltigem Agarmedium.
   - Isolierung der Plasmid-DNA von pUC18-HygR-Mut640
   - DNA-Sequenzanalyse von pUC18-HygR-Mut640
2. Tyr-Codon an Position 1003-1005 (EMBL1.PJHPH) von TAT zu TAG
   Primer 9 (pUC18-HygR-Mut1003) (SEQ ID NO:9)
   - 5'GCAGATCTCCGCGGCTCCGGGCGTAGATGCTCCGC
   - PCR mit Universal-Sequencing-Primer gegen pUC18-HygR-Mut640 Template
   - Restriktion des Amplifikats und pUC18-HygR-Mut640 mit KspI / NheI
   - räparatives Agarosegel; Elution des 240 bp großen Amplifikats und des größeren Fragments von pUC18-HygR-Mut640
   - Ligation von der beiden Fragmente
   - Transformation in E. coli ER1458 (supF) und Selektion auf Hygromycin B (125 µg/ml) haltigem Agarmedium.
   - Isolierung der Plasmid-DNA von pUC18-HygR-Mut640+1003
   - DNA-Sequenzanalyse von pUC18-HygR-Mut640+1003
Überprüfung der beiden Plasmide pUC18-HygR-Mut640 und pUC18-HygR-Mut640+1003 in E. coli ER1562 (supF⁻, supE⁻). Beide Plasmide dürfen keine Hygromycin B-Resistenz mehr vermitteln.

### 3. gegen das Auxotrophie-Markeroperon proAB

Die DNA-Sequenz des proAB-Operons (Deutch, 1984) ist in der EMBL-Datenbank unter EMBL1.ECPROAB gespeichert.
a) zur Klonierung des proAB-Operons mit proB Shine-Dalgarno (SD) Region (Verwendung des Promotors ohne SD von Amp^{R})
   - Amplifikation von pUC18 ohne Amp^{R} und ohne SD mittels PCR Primer 5 (pUC-U2): bp 2500 - 2524 von PUC18.seq; (SEQ ID NO:5) 5'CGAGTGAAGACACCATGGCAATATTATTGAAGCATTTATCAGG Primer 2 (pUC-L): bp 1628 - 1606 von PUC18.seq; (SEQ ID NO:2) 5'GGACTAGATCTGCTAGCTAACTGTCAGACCAAGTTTACTC
   - Restriktion des Amplifikats mit NcoI / NheI
   - Präparatives Agarosegel + Ausschneiden des pUC18-Amplifikats
   - Ligation mit dem Amplifikat des proAB-Operons (s.u.)
   - Amplifikation des proAB-Operons (mit proB SD-Region) aus E. coli K12 Wildtyp-DNA mittels PCR
      Primer 6 (proAB-U): bp 341 - 362 von ECPROAB.EMBL1; (SEQ ID NO:6)
      5'GCTGTAGATCTCCATGGCAGAGAATCATGAGTGAC
      Primer 7 (proAB-L): bp 2692 - 2672 von ECPROAB.EMBL1: (SEQ ID NO:7)
      5'CGACAGATCTGCTAGCTCATTACGCACGAATGGTGTAATC
   - Restriktion des Amplifikats mit NcoI / NheI
   - Präparatives Agarosegel + Ausschneiden des proAB-Amplifikats
   - Ligation mit dem pUC18-Amplifikat (s.o.)
   - Transformation in E. coli JM83 (Δlac-pro)
   - Selektion der proAB⁺-Klone auf Minimalmedium ohne Prolin
   - Isolierung der resultierenden Plasmid-DNA pUC18-proAB(SD)
   - Sequenzanalyse von pUC18-proAB(SD)
b) zur Klonierung des proAB-Operons ohne proB SD-Region (Verwendung von Promotor + SD-Region von Amp^{R})
   - Amplifikation von pUC18 ohne dem Amp^{R}-Leserahmen mittels PCR Primer 1 (pUC-U1) und Primer 2 (pUC-L) (s.o.)
   - Restriktion des Amplifikats mit NcoI / NheI
   - Präparatives Agarosegel + Ausschneiden des pUC18-Amplifikats
   - Ligation mit dem Amplifikat des proAB-Operons (ohne proB SD-Region) (s.u.)
   - Amplifikation des proAB-Operons mit den selben Primem wie unter 2.a) (Primer 6 (proAB-U) und Primer 7 (proAB-L))
   - Restriktion mit BspHI (schneidet stromabwärts der SD-Region direkt am Translationsstart von proB) und NheI
   - Präparatives Agarosegel + Ausschneiden des proAB-Amplifikats
   - Ligation mit dem pUC18-Amplifikat (s.o.)
   - Transformation in E. coli JM83 (Δlac-pro)
   - Selektion der proAB⁺-Klone auf Minimalmedium ohne Prolin
   - solierung der resultierenden Plasmid-DNA pUC18-proAB
   - Sequenzanalyse von pUC18-proAB
ProAB liegt als Operon vor, d.h. beide Gene des proAB-Operons werden von einem Promotor aus transcribiert, zuerst proB (γ-Glutamyl Kinase, GK) dann, stromabwärts proA (γ-Glutamyl-Phosphat Reductase, GPR). GK und GPR formen einen molekularen Komplex, der den direkten Transfer von γ-Glutamyl-Phophat von GK zu GPR sicherstellt (Deutch, 1984). Daher ist das richtige, stöchiometrische Verhältnis von GK zu GPR für die Aktivität des Komplexes wichtig. In einem Operon werden die relativen Expressionsraten der Einzelkomponenten von den Translationsraten der Einzelkomponenten mitbestimmt. Daher wurden zwei Konstrukte des proAB-Operons, einmal mit homologer SD und einmal mit der SD des Amp^{R}-Gens hergestellt.

### 4. Einführen des Inserts aus pCMV-CFTR 936C (Alton, 1993) mit dem CMV-Promotor/Enhancer und Humanen CFTR-Gen in die Sicherheitsvektoren:

- **pUC18-HygR**
- **pUC18-proAB(SD)**
- **pUC18-proAB**
- **pUC18-HygR-Mut640**
- **pUC18-HygR-Mut640+1003**

Isolierung, Reinigung und DNA-Sequenzanalyse der Plasmid-DNA der erhaltenen Konstrukte.
Austestung der erhaltenen Konstrukte im CF-In Vitro-Modell bzw. CF-Mausmodell im Vergleich zu pCMV-CFTR 935C (pUC18-Basis mit Amp^{R}-Gen).

### Literaturverzeichnis:

Alexander, M.Y., Bidichandani, S.I., Robinson, C.J.M. and Akhurst, R.J.: "Treatment of Hemophilia B by Somatic Cell Gene Therapy Using Keratinocytes as a Gene Delivery System." J. of Cellular Biochemistry 18A (1994) Abstract DZ400
Alton, E.W.F.W., Middleton, P.G., Caplen, N.J., Smith, S.N., Steel, D.M., Munkonge, F.M., Jeffery, P.K., Geddes, D.M., Hart, S.L., Williamson, R., Fasold, K.I., Miller, A.D., Dickinson, P., Stevenson, B.J., McLachlan, G., Dorin, J.R. and Porteus, D.J.: "Non-invasive liposome-mediated gene delivery can correct the ion transport defect in cystic fibrosis mutant mice." Nature Genetics 5 (1993) pp.135-142
Arenas, R., Chmura, S.J., Otto, G. and Westbrook, C.A.: "Genetic Modification of Rodent Gut Epithelium by Gene Therapy Using Liposomal Delivery Systems." J. of Cellular Biochemistry 18A (1994) Abstract DZ101
Balows, A., Hauser Jr., W.J., Herrmann, K.L., Isenberg, H.D., Shadomy, H.J.: Manual of Clinical Microbiology, Fifth Edition (1991), American Society for Microbiology, Washington, D.C.
Chiou, H.C., Merwin, J.R., Levine, S.M., Wimler, K.M., Salafia, M.A. and Spitalny, G.L.: "Expression of Secreted and Intracellular Proteins Following Receptor-Mediated Gene Transfer to Hepatocytes In Vivo." J. of Cellular Biochemistry 18A (1994) Abstract DZ109
Cotten, M., Wagner, E., Zatloukal, K., Buschle, M., Chiocca, S., Plank, C., Zauner, W., Schmidt, W. and Birnstiel, M.L.: "Receptor-Mediated Gene Delivery." J. of Cellular Biochemistry 18A (1994) Abstract DZ002
Davis, B.D., Dulbecco, R., Eisen, H.N. and Ginsberg, H.S.: Microbiology, Third Edition (1980), Harper International Edition
Davis, H.L., Demeneix, B.A., Quantin, B., Coulombe, J. and Whalen, R.G.: "Plasmid DNA is Superior to Viral Vectors for Direct Gene Transfer into Adult Mouse Skeletal Muscle." Human Gene Therapy 4 (1993) pp.733-740
Debs, R.J., Zhu, N. and the Regents of the University of California: "Gene Therapy for Cystic Fibrosis Transmembrane Conductance Regulator Activity (CFTR)." WO 93/1224; A1; 17.12.91 US;
Deutch, A.H., Rushlow, K.E. and Smith, C.J.: "Analysis of the Escherichia coli proAB locus by DNA and protein sequencing." NAR 12 (1984) pp.6337-6355
Dodge, J.A., Brock, D.J.H. and Widdicombe, J.H.: Cystic Fibrosis, Current Topics, Volume 1 (1993), John Wiley & Sons
FitzSimmons, S.C.: "The changing epidemiology of cystic fibrosis." The Journal of Pediatrics 122 (1993) pp.1-9
Gao, L., Wagner, E., Cotten, M., Agarwal, C., Harris, C., Romer, M., Miller, L., Hu, P.-C. and Curiel, D.: "Direct In Vivo Gene Transfer to Airway Epithelium Employing Adenovirus-Polylysine-DNA Complexes." Human Gene Therapy 4 (1993) pp.17-24
Gritz, L. and Davies, J.: "Plasmid-encoded hygromycin B resistance: the sequence of hygromycin B phosphotransferase gene and its expression in Escherichia coli and Saccharomyces cerevisiae." Gene (1983) pp.179-188
Hunter, J.E.B., Shelley, J.C., Walton, J.R., Hart, C.A. and Bennett, M.: "Apramycin resistance plasmids in Escherichia coli: possible transfer to Salmonella typhimurium in calves." Epidemiol. Infect. 108 (1992) pp.271-278
Hyde, S.C., Gill, D.R., Higgins, C.F., Trezise, A.E.O., MacVinish, L.J., Cuthbert, A.W., Ratcliff, R., Evans, M.J. and Colledge, W.H.: "Correction of the ion transport defect in cystic fibrosis transgenic mice by gene therapy." Nature 362 (1993) pp.250-255
Jimenez, A. and Davies, J.: "Expression of a transposable antibiotic resistance element in Saccharomyces." Nature 287 (1980) pp.869
Kamakura, T., Kobayashi, K., Tanaka, T., Yamaguchi, I. and Endo, T.: "Cloning and Expression of a New Structural Gene for Blasticidin S Deaminase, a Nucleoside Aminohydrolase." Agric. Biol. Chem 51 (1987) pp.3165-3168
Lew, D. and Latimer, T.: "Long Term Persistence of Plasmid DNA after Intramuscular Injection in Mice." J. of Cellular Biochemistry 18A (1994) Abstract DZ120
Lori, F., Lisziewicz, J., Smythe, J., Cara, A., Bunnag, T.A., Curiel, D. and Gallo, R.C.: "Rapid protection against human immunodeficiency virus type 1 (HIV-1) replication mediated by high efficiency non-retroviral delivery of genes interfering with HIV-1 tat and gag." Gene Therapy 1 (1994) pp.27-31
Morgan, R.A. and Anderson, W.F.: "Human Gene Therapy." Annu. Rev. Biochem. 62 (1993) pp.191-217
Mulsant, P., Gatignol, A., Dalens, M. and Tiraby, G.: "Phleomycin Resistance as a Dominant Selectable Marker in CHO Cells." Somatic Cell and Mol. Genetics 14 (1988) pp.243-252
Nabel, G.I., Nabel, E.G., Yang, Z.-Y., Fox, B.A., Plautz, G.E., Gao, X., Huang, L., Gordon, D. and Chang, A.E.: "Direct gene transfer with DNA-liposome complexes in melanoma: Expression, biologic activity, and lack of toxicity in humans." PNAS USA 90 (1993) pp.11307-11311
Nabel, G.J., Fox, B.A., Post, L., Thompson, C.B., Woffendin, C.: "Clinical Protocol: A Molecular Genetic Intervention for AIDS - Effects of a Transdominant Negative Form of Rev." Human Gene Therapy 5 (1994) pp.79-92
Newbold, C.J., Wallace, R.J. and Watt, N.D.: "Properties of Ionophore-resistant Bacterioides ruminicola enriched by cultivation in the presence of tetronasm". J. of Appl. Bacteriol. 72 (1992) pp.65-70
Recombinant Advisory Committee (RAC) DATA MANAGEMENT Report - June 1993; Human Gene Therapy 5 (1994) pp.135-136
Rote Liste 1994, Arzneimittelverzeichnis des BPI, ECV, Aulendorf/Württ.
San, H., Yang, Z.-Y., Pompili, V.J., Jaffe, M.L., Plautz, G.E., Xu, L., Felbner, J., Wheeler, C.J., Felgner, P.L., Gao, X., Huang, L., Gordon, D., Nabel, G.J. and Nabel, E.G.: "Safety and Short-Term Toxicity of a Novel Cationic Lipid Formulation for Human Gene Therapy." Human Gene Therapy 4 (1993) pp.781-788
Seed, B.: "Purification of genomic sequences from bacteriophage libraries by recombination and selection in vivo." NAR (1983) pp.2427-2445
Ulmer, J.B., Donnelly, J.J., Parker, S.E., Rhodes, G.H., Felgner, P.L., Dwarki, V.J., Gromkowski, S.H., Deck, R.R., DeWitt, C.M., Friedman, A., Hawe, L.A., Leander, K.R., Martinez, D., Perry, H.C., Shiver, J.W., Montgomery, D.L. and Liu, M.A.: "Heterologous Protection Against Influenza by Injection of DNA Encoding a Viral Protein." Science 259 (1993) pp.1745-1749
Wagner, E., Zenke, M., Cotten, M., Beug, H. and Birnstiel, M.L.: "Transferrin-polycation conjugates as carriers for DNA uptake into cells." PNAS USA 87 (1990) pp.3410-3414
Whitsett, J.A., Dey, C.R., Stripp, B.R., Wikenheiser, K.A., Clark, J.C., Wert, S.E., Gregory, R.J., Smith, A.E., Cohn, J.A., Wilson, J.M and Engelhardt, J.: " Human cystic fibrosis transmembrane conductance regulator directed to respiratory epithelial cells of transgenic mice." Nature Genetics 2 (1992) pp.13-20
Yoshimura, K., Rosenfeld, M.A., Nakamura, H., Scherer, E.M., Pavirani, A., Lecocq, J.-P. and Crystal, R.G.: "Expression of the human cystic fibrosis transmembrane conductance regulator gene in the mouse lung after in vivo intratracheal plasmid-mediated gene transfer." NAR 20 (1992) pp.3233-3240
Zabner, J., Couture, L.A., Gregory, R.J., Grahem, S.M., Smith, A.E. and Welsh, M.J.: "Adenovirus-Mediated Gene Transfer Transiently Corrects the Chloride Transport Defect in Nasal Epithelia of Patients with Cystic Fibrosis." Cell 75 (1993) pp.207-216
Zakharyan, R.A., Gasparyan, E.T. and Aposhyan, G.V.: "Transport of a β-lactamase gene into human cells by artificial virus-like particles and its expression." Biol. Zh. Arm. (1982) pp.730-734

SEQUENZPROTOKOLL

(1) ALGEMEINE INFORMATION:

(i) ANMELDER:
(A) NAME: BOEHRINGER MANNHEIM GMBH
(B) STRASSE: Sandhofer Str. 116
(C) ORT: Mannheim
(D) BUNDESLAND: BW
(E) LAND: Germany
(F) POSTLEITZAHL: D-68305
(G) TELEPHON: 08856/60-3446
(H) TELEFAX: 08856/60-3451

(ii) ANMELDETITEL: Gentherapeutisches Verfahren
unter Verwendung von antibiotikaresistenz-
genfreien DNA-Vektoren

(iii) ANZAHL DER SEQUENZEN: 9

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version
#1.25 (EPA)

(vi) FRÜHERE ANMELDEDATEN:
(A) ANMELDENUMMER: DE P 44 10 188.0
(B) ANMELDEDATUM: 24-MAR-1994

(2) INFORMATION ZU SEQ ID NO: 1:

(i) SEQUENZ CHARAKTERISTIKA:
(A) LÄNGE: 43 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 1:

CGAGTGAAGA CACCATGGTC TTCCTTTTTC AATATTATTG AAG 43

(2) INFORMATION ZU SEQ ID NO: 2:

(i) SEQUENZ CHARAKTERISTIKA:
(A) LÄNGE: 40 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 2:

GGACTAGATC TGCTAGCTAA CTGTCAGACC AAGTTTACTC 40

(2) INFORMATION ZU SEQ ID NO: 3:

(i) SEQUENZ CHARAKTERISTIKA:
(A) LÄNGE: 39 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 3:

GCTGTAGATC TCATGAAAAA GCCTGAACTC ACCGCGACG 39

(2) INFORMATION ZU SEQ ID NO: 4:

(i) SEQUENZ CHARAKTERISTIKA:
(A) LÄNGE: 40 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 4:

CGACAGATCT GCTAGCTCAT TATTCCTTTG CCCTCGGACG 40

(2) INFORMATION ZU SEQ ID NO: 5:

(i) SEQUENZ CHARAKTERISTIKA:
(A) LÄNGE: 43 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 5:

CGAGTGAAGA CACCATGGCA ATATTATTGA AGCATTTATC AGG 43

(2) INFORMATION ZU SEQ ID NO: 6:

(i) SEQUENZ CHARAKTERISTIKA:
(A) LÄNGE: 35 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 6:

GCTGTAGATC TCCATGGCAG AGAATCATGA GTGAC 35

(2) INFORMATION ZU SEQ ID NO: 7:

(i) SEQUENZ CHARAKTERISTIKA:
(A) LÄNGE: 40 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 7:

CGACAGATCT GCTAGCTCAT TACGCACGAA TGGTGTAATC 40

(2) INFORMATION ZU SEQ ID NO: 8:

(i) SEQUENZ CHARAKTERISTIKA:
(A) LÄNGE: 44 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 8:

GCAGATCTCG GACCGCAAGG AATCGGTCAA TAGACTACAT GGCG 44

(2) INFORMATION ZU SEQ ID NO: 9:

(i) SEQUENZ CHARAKTERISTIKA:
(A) LÄNGE: 35 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 9:

GCAGATCTCC GCGGCTCCGG GCGTAGATGC TCCGC 35

## Patentansprüche

1. Verwendung einer Vektor-DNA zur Herstellung eines Arzneimittels zur gentherapeutischen Behandlung von Säugetieren oder Menschen, wobei die Vektor-DNA eine Modulation, Korrektur oder Aktivierung der Expression eines endogenen Gens oder die Expression eines durch die Vektor-DNA in die Zellen des Säugetieres oder des Menschen eingeführten Gens bewirkt, dadurch gekennzeichnet, daß die Vektornukleinsäure kein aktives Antibiotikaresistenzgen enthält.

2. Verwendung einer Vektor-DNA zur Herstellung eines Arzneimittels zur gentherapeutischen Behandlung von Menschen, wobei die Vektor-DNA eine Modulation, Korrektur oder Aktivierung der Expression eines endogenen Gens oder die Expression eines durch die Vektor-DNA in die Zellen des Menschen eingeführten Gens bewirkt, dadurch gekennzeichnet, daß die Vektornukleinsäure ein Resistenzgen für Hygromycin, Chloramphenicol, Spectinomycin, Blasticidin S, Phleomycin, Bleomycin, Puromycin, Apramycin oder Tetronasin enthält.

3. Verwendung nach Anspruch 1 oder 2, dadurch gekennzeichnet, daß die gentherapeutische Behandlung in den Atmungswegen, in dem Verdauungstrakt oder auf der Hautoberfläche erfolgt.

4. Verwendung nach den Ansprüchen 1 bis 3, dadurch gekennzeichnet, daß die Vektor-DNA eine Modulation oder Aktivierung der Expression des endogenen Gens für die zystische Fibrose bewirkt oder für das zystische Fibrosegen codiert.

5. Verwendung nach Anspruch 4, dadurch gekennzeichnet, daß das Arzneimittel als Aerosol zur Anwendung im Atmungstrakt hergestellt wird.

6. Vektor-DNA, welche ein Gen oder Genfragment enthält, das die Modulation, Aktivierung oder Korrektur eines endogenen zystischen Fibrosegens in Säugerzellen bewirkt, oder welche ein in Säugerzellen exprimierbares zystisches Fibrosegen enthält, dadurch gekennzeichnet, daß diese Vektor-DNA ein inaktiviertes Antibiotikaresistenzgen oder ein Gen, welches für einen auxotrophen Marker codiert, enthält.

7. Vektor-DNA, welche ein Gen oder Genfragment enthält, das die Modulation, Aktivierung oder Korrektur eines endogenen zystischen Fibrosegens in Säugerzellen bewirkt, oder welche ein in Säugerzellen exprimierbares zystisches Fibrosegen enthält, dadurch gekennzeichnet, daß diese Vektor-DNA ein Resistenzgen für Hygromycin, Chloramphenicol, Spectinomycin, Blasticidin S, Phleomcin, Bleomycin, Puromycin, Apramycin oder Tetronasin enthält.
